# EUROPEAN PATENT APPLICATION

(11) **EP 3 466 510 A1**
(43) Date of publication of application: **10.04.2019**
(21) Application number: 17194925.8
(22) Date of filing: 05.10.2017
(51) Int. Cl.: B01D 11/02, C07K 1/14, C11B 1/10

(54) **METHOD FOR DETECTING PESTICIDES IN FOOD PRODUCTS**

(71) Applicant: NofaLab B.V., 3315 JG Schiedam (NL)
(72) Inventor: BROER, Wilhelmus, 3079 ZC Rotterdam (NL)
(74) Representative: van Kooij, Adriaan

(57) **Abstract**

The present invention relates to a method for the detection of pesticides in food products. More particularly, the present invention relates to the detection of pesticides in food products by means of liquid chromatography and wherein the method comprises the use of an extraction liquid comprising a mixture of water (H₂O), methanol (CH₃OH) and formic acid (CH₂O₂), and an a-polar solvent. Furthermore, the present invention relates to the method wherein an additional purification step is carried out by means of solid phase extraction under vacuum.

## Description

The present invention relates to a method for the detection of pesticides in food products. More particularly, the present invention relates to the detection of pesticides in food products by means of liquid chromatography, and wherein the method comprises the use of an extraction liquid comprising a mixture of water (H₂O), methanol (CH₃OH) and formic acid (CH₂O₂), and an a-polar solvent. Furthermore, the present invention relates to the method wherein an additional purification step is performed by means of solid phase vacuum extraction.

Given the continuous growth of the world population, the demand for food is constantly growing. For the production of our food is often made use of various chemicals to ensure the quality and improve our food but also to increase the quantity of our food. Extensive use of chemical agents such as pesticides (insecticides, fungicides, herbicides) is adopted to control insects, weeds, microbes, etc. in order to improve the conditions for growth of vegetables and fruit and increase crop yield. The mode of action and toxicity of these agents used vary greatly and their use is not uncontroversial, since these substances can contaminate our food and there is a risk that these chemicals end up and build up in our bodies and therefore may pose a health risk.

The physical and chemical characteristics of pesticides can be very divers. For example, there are a-polar pesticides which are insoluble in water, moderately polar to polar pesticides which are partly to completely soluble in water and highly-polar and ionic pesticides which are sparingly soluble in an organic solvent to completely soluble in water.

In recent years a lot of effort has been put in the establishment and development of efficient, robust and sensitive analytical methods for the detection and determination of pesticides that are present in our food. Commonly used methods for analysing chemicals in food are for instance Gas Chromatography (GC) and Liquid Chromatography (LC). Chromatography has made a strong advance in recent years, wherein the chemicals are separated in a gas (by GC) or liquid (LC) phase on their selective distribution between the stationary (often a column or matrix), and the mobile phase (a carrier gas (GC) or eluent (LC)). The substances that need to be separated or identified are carried along by the carrier gas or eluent and interact with the column phase, in which molecules are bonded temporarily to the column, depending on the affinity of the molecules of the substance with the column phase. As a result, it is possible to separate substances from each other. Subsequently, the detection of the substances takes place by means of various detectors, such as, for example, mass spectrometry.

However, chromatography is very sensitive to matrix effects, i.e. substances other than where one is interested in that are present in the sample, resulting in that systems are able to optimally measure only for a short period of time. Current technologies and methods allow for stable and reliable measurements with respect to the detection of pesticides in for example food from about one to a few days maximum. After this period, the columns and detector will become contaminated and/or clogged and are unusable for efficient and sensitive detection of chemicals and should either be cleaned or replaced. Therefore, it is currently of interest to inject on the column as little as possible of the extracts / samples. This can be achieved by means of extensive purification of the samples by means of extraction and / or diluting these samples, which can be a time-consuming undertaking. Bottleneck with such a diluent or purified sample is to achieve detection within the desired detection limits, in which the detection limits for chemicals in foods, such as insecticides and herbicides, continues to be lowered (adjusted) in order to monitor food safety and protect the health of the consumer.

For the extraction of pesticides an extraction liquid is used comprising a mixture of water, alcohol (e.g. methanol), an organic cyanide (e.g. acetonitrile), and an organic acid (e.g. formic acid). Depending on the polarity of a pesticide, an extraction method may be selected. For example, the a-polar to polar pesticides can be extracted from the food product with a salt-saturated aqueous buffer and an organic solvent that is immiscible with water. The buffer should regulate the pH during the extraction and should make the polar pesticides medium-soluble in water, in order to achieve that all the pesticide after extraction will be present in the organic solvent. The extract is further purified before the analysis is carried out.

However, for the extraction of highly polar and ionic pesticides (or chemicals) the previously described extraction method does not work, and an aqueous extraction is more likely to be used because these compounds are all very well soluble in water. However, this extraction often results in suspensions that are difficult to separate into an aqueous portion and solid portion, and in the end, no stable chromatography will be obtained. In addition, there are many proteins, sugars, carbohydrates and fatty acids present in the extract which adversely affect the life time of the columns. As a result of this it was shown from the chromatographic analysis of the extract that many disturbing matrix is present through which retention times shifted from maximally a minute to more than a few minutes.

Another disadvantage of current methods for the detection and/or determination of these pesticides in food products is that they are often only suitable for one or more similar analytes. For example, glyphosate can be measured after a time-consuming derivatization process, and chlorate and perchlorate are measured by means of elaborate ion chromatography. In addition, known methods are mainly only suitable for aqueous samples.

Furthermore, most chromatography columns are often developed and suitable for use with water samples, in which no account is taken of the large amount of organic co-extracts, that arise as a result of the processing of the samples derived from foods. These organic co-extracts accumulate on to the column, causing the column to degenerate, resulting in a poor separation and reduced quality of the chromatographic analysis and detection of chemicals (i.e. the insecticides and herbicides). The end result is that the chromatography columns are rapidly "aging", the sensitivity of the columns fluctuates, and the retention time varies, which adversely affects an efficient and robust detection of pesticides. In addition, the maximum residue limit (MRL), which indicates how much pesticide may be left in or on the food product in recent years has been reduced from 500 µg/kg to 10-30 µg/kg. Thus a very sensitive detection method is necessary. To achieve this, presently the highly polluted chromatographic columns are to be regenerated or replaced frequently.

In view of the above, there is a need in the art for a more robust, efficient and sensitive method for the detection and/or determination of chemicals such as highly polar and ionic pesticides in food products, and to prolong the life time of the chromatographic columns and to reduce matrix effects. A further object of the present invention is to provide an increased detection of a wide range of polar pesticides such as diquat, paraquat, glyphosate, glufosinate, ethephon, fosetyl, chlorate, perchlorate, and their metabolites by means of one extraction method, and preferably a single chromatographic method of analysis.

It is an objective of the present invention, amongst other objects, to address the above need in the art. The object of present invention, amongst other objects, is met by the present invention as outlined in the appended claims.

In particular, the above object is, amongst others, according to a first aspect achieved according to the present invention by a method for the detection of pesticides in food products and / or raw materials thereof, wherein the method comprises the following steps;
a) contacting of a sample with an extraction liquid comprising a mixture of water (H₂O), methanol (CH₃OH) and formic acid (CH₂O₂), and mixing to form a suspension,
b) homogenizing the suspension followed by maintaining the temperature of the suspension at about 70 °C to 80 °C for at least 10 minutes,
c) allowing the suspension to cool down,
d) contacting and mixing of the suspension from step c with an a-polar solvent,
e) homogenizing the suspension,
f) centrifuging the suspension of step e,
g) isolating a water/methanol fraction and a solvent fraction,
h) detecting pesticides in the solvent fraction by means of liquid chromatography.

With the method of the present invention, an improved purified extract is obtained and therefore less sample can be used with increased detection sensitivity. In other words, the sample is further optimized and less sample can be used during liquid chromatography and at the same time to continue to measure within the pre-set (and continues lowered) detection limits. In addition, the method according to the present invention has led to the fact that instead of stable detection was possible for only one day with Liquid Chromatography, stable detection is now possible for more than a week. LC Columns get a lot less polluted, and thus last longer, resulting in an improvement in the detection of pesticides in both quantity and quality (detection sensitivity, detection rate).

Examples of food products and / or raw materials that can be analysed by the method of the present invention are fruits, vegetables, dried herbs such as chili powder, nutmeg, rapeseed, sunflower seed, sunflower oil, rapeseed oil, cereals, meat products such as minced meat and chicken fillet, milk powder, cheese, eggs, fish oil and fatty acids of animal or vegetable origin.

According to a preferred embodiment, the present invention relates to the method wherein the ratio of water, methanol and formic acid in the extraction liquid is selected from the group consisting of 60:39:1, 49.5:49.5:1, 54:45:1, preferably 60:39:1. It has been investigated which extraction fluid results in optimal extraction of the highly polar pesticides. Highly polar pesticides are ionic pesticides which are highly soluble in water. However, to make the sample more accessible, methanol and formic acid are added to the extraction liquid. Eventually, a mixture of water, methanol and formic acid in the ratio of 60:39:1 seems to be most optimal. The extraction liquid as described in the method of the present invention results in a reduction in the occurrence of difficult to dissolve suspensions and reduces the number of contaminants such as organic co-extracts present in the sample to be analysed after extraction, such as the presence of proteins and sugars.

According to another preferred embodiment, the present invention relates to the method wherein the weight ratio of sample: extraction liquid in the suspension of step a) is at least about 1:5, preferably at least about 1:10, most preferably at least about 1:20.

According to yet another preferred embodiment, the present invention relates to the method wherein the suspension in step b) is heated to between 72 °C to 76 °C, preferably to about 73 °C. In order to facilitate extraction of the pesticides from the sample and to improve the "availability" of the pesticide in order to achieve an extraction as efficient as possible, the suspension is heated to between 72 °C and 76 °C. At these temperatures, the uptake of the compounds (pesticides) in the H₂O/CH₃OH phase is improved as is the extraction efficiency. The most optimal extractions of pesticides were obtained at about 73 °C.

According to a preferred embodiment, the present invention relates to the method wherein the suspension in step b) is heated for at least 15 minutes, preferably for at least 20 minutes.

According to yet another preferred embodiment, the present invention relates to the method wherein the a-polar solvent is selected from the group consisting of dichloromethane, hexane, chloroform or difluoromethane, preferably dichloromethane. Before the a-polar solvent is added to the suspension of step b, the suspension is cooled to at least below the boiling point of the a-polar solvent, preferably to between 15 °C and 50 °C, more preferably to at least room temperature. The added solvent breaks the formed suspension and ensures that the organic oils and fats which are present in the sample will migrate to the solvent, dichloromethane is preferably used because it provides the best results in terms of purification in greasy samples and additionally can be obtained as the most clean solvent on the market.

According to another preferred embodiment, the present invention relates to the method wherein said pesticides are selected from the group consisting of glyphosate, diquat, paraquat, aminomethylphosphonic acid (AMPA), N-acetyl-glufosinate, glufosinate, metabolite 3-methylfosfinicopropionic acid (3-MPPA), phosphonic acid, fosetyl, chlorate, ethephon, perchlorate, preferably glyphosate.

According to a preferred embodiment, the present invention relates to the method wherein when said sample relates to a solid substance, the solid substance has a particle size of at most 500 µm, preferably at most 300 µm.

According to yet another preferred embodiment, the present invention relates to the method wherein after step f) an additional purification step is carried out, i) extraction under vacuum by means of solid phase extraction (SPE) with at least two columns.

According to a preferred embodiment, the present invention relates to the method wherein one of the at least two columns is a graphite-carbon based column, and one of the at least two columns is a C18 column. The columns serve as a further purification and removal of compounds that are not soluble in water and colorants present in the sample.

According to another preferred embodiment, the present invention relates to the method wherein extraction is carried out over the columns with at least one drop per second.

According to yet another preferred embodiment, the present invention relates to the method wherein said detection of pesticides in food products and/or raw materials thereof comprises a maximum residue limits (MRL) of at most 500 µg/kg, preferably of at most 50 µg/kg, most preferably of at most 10 µg/kg.

The present invention will be further detailed in the following examples;

### Sample preparation

Solid samples shall first be reduced by grinding for example with a grinder to obtain a particle size of less than 500 µm.

### Extraction

The equivalent of 1 ± 0.1 g of sample is taken into consideration. In the case of a dried product, the original water content in the product will be taken into account. See table 1 for the weigh of the samples.

**Table 1: Weighing and extraction fluid**

| **Product** | **Sample (g)** | **Extraction Liquid (ml)** |
|---|---|---|
| Fatty acids | 1 | 20 |
| Oils and / or fats | 1 | 20 |
| Dried solid products and spices. | 1 | 20 |
| Baby food | 5 | 20 |

Add to the sample, after the weighing, 20 ml of the extraction liquid MeOH:H₂O: FA, 39:60:1. Manually shake the tube immediately after the addition of the extraction liquid, and extract the sample, then vortex at least for 5 minutes. Then, place the samples for 20 minutes in a water bath at 73 ± 0.2 °C and subsequently let to cool down to room temperature. After cooling, add 5 ml of dichloromethane to the suspension and vortex it once again for at least 5 minutes on the multi-vortex. Centrifuge the sample for at least 5 minutes at 3.500g to 5.000g, preferably at 4.000g.

For the analysis of the most polar pesticides, a further purification step is performed with the vacuum SPE-system, after centrifugation. The vacuum SPE system is before use cleaned with Demi / RO water. Firstly, place the Carbon SPE with on top the C18 SPE on the vacuum SPE system. Then transfer 3 ml of extract onto the C18 SPE. Use a 15 mL centrifuge tube as a collecting tube and apply as much vacuum to the system SPE- to achieve a flow through of one drop per second. Transfer the extract into a PE-vial for LCMS analysis.

### Chromatographic analysis of the extracts obtained from glyphosate, aminomethylphosphonic acid (AMPA), N-acetyl-glufosinate, glufosinate, metabolite 3-methylfosfinicopropionic acid (3-MPPA), phosphonic acid, fosetyl, chlorate, ethephon.

The analysis of the extracts is performed by liquid chromatography (LC) which is coupled to a triple quadrupole mass spectrometer. For the chromatographic separation, use is made of column filled with a column of material of polyvinyl alcohol with quaternary ammonium groups (Metrohm, MetrosepA Supp 5150 * 4 mm). The column is protected with a guard column of the same type, and a particle filter of 0.5 µm. The eluent consists of a buffer of ammonium bicarbonate at a pH of from about 8.8 to 9.0 in an ion-strength controlled gradient program.

## Claims

1. Method for the detection of pesticides in food products and / or raw materials thereof, wherein the method comprises the following steps;
a) contacting of a sample with an extraction liquid comprising a mixture of water (H₂O), methanol (CH₃OH) and formic acid (CH₂O₂), and mixing to form a suspension,
b) homogenizing the suspension followed by maintaining the temperature of the suspension at about 70 °C to 80 °C for at least 10 minutes,
c) allowing the suspension to cool down,
d) contacting and mixing of the suspension from step c with an a-polar solvent,
e) homogenizing the suspension,
f) centrifuging the suspension of step e,
g) isolating a water/methanol fraction and a solvent fraction,
h) detecting pesticides in the solvent fraction by means of liquid chromatography.

2. A method according to claim 1, wherein the ratio of water, methanol and formic acid in the extraction liquid is selected from the group consisting of 60:39:1, 49.5:49.5:1, 54:45:1, preferably 60:39:1.

3. A method according to claim 1 or 2, wherein the weight ratio of sample: extraction liquid in the suspension of step a) is at least about 1:5, preferably at least about 1:10, most preferably at least about 1:20.

4. A method according to any one of claims 1 to 3, wherein the suspension in step b) is heated to between 72 °C to 76 °C, preferably to about 73 ° C.

5. A method according to any one of claims 1 to 4, wherein the suspension in step b is heated for at least 15 minutes, preferably for at least 20 minutes.

6. A method according to any one of claims 1 to 5, wherein the a-polar solvent is selected from the group consisting of dichloromethane, hexane, chloroform or difluoromethane, preferably dichloromethane.

7. A method according to any one of the preceding claims, wherein said pesticides are selected from the group consisting of glyphosate, diquat, paraquat, aminomethylphosphonic acid (AMPA), N-acetyl-glufosinate, glufosinate, metabolite 3-methylfosfinicopropionic acid (3-MPPA), phosphonic acid, fosetyl, chlorate, ethephon, perchlorate.

8. A method according to any one of the preceding claims, wherein when said sample relates to a solid substance, the solid substance has a particle size of at most 500 µm, preferably at most 300 µm.

9. A method according to any one of the preceding claims, wherein after step f) an additional purification step is carried out, i) extraction under vacuum by means of solid phase extraction (SPE) with at least two columns.

10. A method according to claim 9, wherein one of the at least two columns is a graphite-carbon based column, and one of the at least two columns is a C18 column.

11. A method as claimed in claim 9 or 10, wherein extraction is carried out over the columns with at least one drop per second.

12. A method according to any one of the preceding claims, wherein said detection of pesticides in food products and/or raw materials thereof comprises a maximum residue limits (MRL) of at most 500 µg/kg, preferably of at most 50 µg/kg, most preferably of at most 10 µg/kg.
